Europäisches Patentamt

(19) **European Patent Office**

Office européen des brevets

(11) Veröffentlichungsnummer: **0 009 812**
**A1**

(12) **EUROPÄISCHE PATENTANMELDUNG**

(21) Anmeldenummer: **79103789.8**

(22) Anmeldetag: **04.10.79**

(51) Int. Cl.³: **C 07 C 69/15**
**C 07 C 67/60**

(30) Priorität: **05.10.78 DE 2843464**

(43) Veröffentlichungstag der Anmeldung:
**16.04.80 Patentblatt 80/8**

(84) Benannte Vertragsstaaten:
**AT BE CH DE FR GB IT NL**

(71) Anmelder: **WACKER-CHEMIE GMBH**
**Prinzregentenstrasse 22**
**D-8000 München 22(DE)**

(72) Erfinder: **Rostock, Kai, Dr. Dipl.-Chem.**
**Seitenhornstrasse 11**
**D-8263 Burghausen(DE)**

(72) Erfinder: **Birke, Hans**
**Bruckbergstrasse 19**
**D-8261 Marktl/Inn(DE)**

(72) Erfinder: **Radecker, Josef**
**Fischerweg 13**
**D-8261 Emmerting(DE)**

(72) Erfinder: **Bauer, Hans, Dr. Dipl.-Chem.**
**Görresstrasse 3**
**D-8263 Burghausen(DE)**

(72) Erfinder: **Stange, Hans, Dr. Dipl.-Chem.**
**Hohenbrunner Strasse 3**
**D-8000 München 82(DE)**

(54) **Verfahren zur Abtrennung von Aldehyden aus Vinylacetat.**

(57) Verfahren zur Abtrennung von Aldehydverunreinigungen aus Vinylacetat, indem man dieses mit einer wässerigen
Natrimhydrogensulfitlösung vermischt, so den Aldehyd in die
wässerige Phase überführt und diese mischt, anschließend
abtrennt. Es wird so Vinylacetat ohne aldehydische Verunreinigung erhalten, das zur radikalischen Polymerisation zur
Herstellung stabiler Polymerisate geeignet ist.

BAD ORIGINAL

WACKER-CHEMIE
GMBH

München, den 25.8.1978
LC-PAT/Dr.St/we

Wa 7841-V
=========

- 1 -

## Verfahren zur Abtrennung von Aldehyden aus Vinylacetat

Gegenstand der Erfindung ist ein Verfahren zur Reinigung von Vinylacetat von darin enthaltenen Aldehyden, insbesondere Acetaldehyd.

Vinylacetat wird vorwiegend durch Polymerisation in Homo- bzw. Copolymerisate umgewandelt. Bei der radikalischen Polymerisation,
die in Masse, Lösung oder Dispersion erfolgen kann, stören die im
Monomeren anwesenden Aldehyde in der Weise, daß sie regelnd auf
den Polymerisationsprozeß einwirken, d.h. zu vorzeitigem Kettenabbruch im Polymerisationsprozeß führen. Aldehydkonzentrationen
über 50 ppm sind dabei schon merklich wirksam. Durch die Anwesenheit von Aldehyden während der Polymerisation verschlechtert sich
außerdem die Stabilität des Polymerisationsproduktes.

Aldehyde, insbesondere Acetaldehyd, können sowohl vom Produktionsprozeß her eingeschleppt werden, oder Acetaldehyd entsteht aus
Vinylacetat durch Hydrolyse. Die destillative Trennung von Aldehyden und Vinylacetat, insbesondere Acetaldehyd, gestaltet sich
schwierig und konnte nur mit Kolonnen mit unwirtschaftlich hoher
Bodenzahl gelöst werden.

Aldehyde zeichnen sich dadurch aus, daß sie sich mit einer Reihe reaktiver Verbindungen zu Additionsprodukten vereinen. So sind beispielsweise die Additionsreaktionen der Aldehyde mit Phenylhydrazin, Anilin, Harnstoff, Hydrazin, Hydroxylamin und Natriumhydrogensulfit bekannt. Diese werden als Gleichgewichtsreaktionen beschrieben. Bisher ist jedoch kein Hinweis bekannt, in welcher Weise Aldehyde aus dem ebenfalls reaktionsfreudigen Vinylacetat praktisch quantitativ, d.h. bis auf Restmengen unter 50 ppm Aldehyd im Vinylacetat entfernbar wären.

Zweck der Erfindung ist es, Vinylacetat für Polymerisationszwecke mit möglichst niedrigem Aldehydgehalt zur Verfügung zu stellen.

Aufgabe der Erfindung ist es, ein Verfahren zur Abtrennung von Aldehyden, insbesondere Acetaldehyd, aus Vinylacetat anzugeben, bei dem die Aldehydverunreinigung aus einem großen Überschuß Vinylacetat bis auf eine Restkonzentration unter 50 ppm auf einfache Weise abgetrennt werden kann.

Gegenstand der Erfindung ist ein Verfahren zur Abtrennung von Aldehyden, insbesondere Acetaldehyd, aus Vinylacetat, wobei das zu reinigende Vinylacetat mit bis zu $5 \times 10^4$ ppm Aldehyd, insbesondere Acetaldehyd, verunreinigt ist und das gereinigte Vinylacetat nach der Reinigung weniger als 50 ppm Aldehyd aufweist, dadurch gekennzeichnet, daß das zu reinigende Vinylacetat mit Alkalihydrogensulfit in wäßriger Lösung, vorzugsweise der äquimolaren Menge Natriumhydrogensulfit in wäßriger Lösung, bezogen auf Aldehyd, durchmischt wird und nach erfolgter Phasentrennung die wäßrige Phase abgelassen wird. Mit dem erfindungsgemäßen Verfahren ist es möglich, Aldehyde kontinuierlich und auch diskontinuierlich aus Vinylacetat abzutrennen.

Auch der Zusatz von pH-stabilisierenden Salzen oder auch Neutralsalzen zur Beschleunigung der Phasentrennung sowie Entschäumungsmittel können einzeln oder in Kombination zugesetzt werden.

Das Verfahren ist besonders dann günstig, wenn die wäßrige Phase nach der Phasentrennung einen pH-Wert zwischen 4 und 8 aufweist. Ebenso ist es vorteilhaft, das Volumen der wäßrigen Phase so auszuwählen, daß es 0,5 bis 35 % des Volumens des zu reinigenden Vinylacetats ausmacht. Die Natriumhydrogensulfitlösung hat dabei Konzentrationen zwischen 1 und 35 Gew.%.

Überraschenderweise wird es durch das erfindungsgemäße Verfahren möglich, Aldehyde und insbesondere Acetaldehyd praktisch quantitativ, d.h. bis zu Restkonzentrationen unterhalb 50 ppm aus dem Vinylacetat in die wäßrige Phase zu überführen. Dies ist um so erstaunlicher, da man bei Kenntnis der herrschenden Lehrbuchmeinung davon ausgehen mußte, daß sich ein Gleichgewicht zwischen Natriumhydrogensulfitadditionsverbindung und Acetaldehyd einstellt. Zudem war es nicht zu erwarten, daß das im wäßrigen Medium behandelte Vinylacetat unter den Verfahrensbedingungen nicht der Hydrolyse unterliegt und auch nach Entfernung der wäßrigen Phase und ggf. Waschen mit Wasser oder Salzlösungen ein Vinylacetat mit für die Polymerisation hinreichend niedrigen Aldehydgehalten verfügbar wird.

Vinylacetat enthält aufgrund des Herstellungsprozesses häufig Acetaldehyd. Aber auch bei der Lagerung oder auch bei der Polymerisation von Vinylacetat zu Homo- oder Copolymerisaten entsteht aus Vinylacetat durch Hydrolyse Acetaldehyd, der dann bei der Monomerrückgewinnung nach der Polymerisation gemeinsam mit den nicht umgesetzten Monomeren anfällt. Dieser Acetaldehyd kann durch Adolkondensation zu höheren Aldehyden kondensieren. Aldehydhaltiges Vinylacetat kann jedoch nicht ohne Nachteile bei anschließender Wiederverwendung in Kauf nehmen zu müssen, einer erneuten Polymerisation unterworfen werden.

Eine typische Zusammensetzung eines Restmonomerkondensates, wie es bei der an die Copolymerisation von Vinylchlorid/Vinylacetat anfällt, hat folgende Zusammensetzung: 1,5 Gew.% Wasser, 0,5 Gew.% Acetaldehyd, 3 Gew.% Vinylchlorid, 1 Gew.% Trichloräthylen und 94 Gew.% Vinylacetat. Alle Begleitkomponenten des Vinylacetats

außer den Aldehyden können durch einfache Destillation entfernt werden. Die Entfernung der Aldehyde ist jedoch durch Destillation nur schwierig zu erreichen. Selbst bei einem hohen Rücklaufverhältnis (10 : 1) und Verwerfung von 10 Gew.% Vinylacetat als Vorlauf, verbleiben 1000 ppm Aldehyd in der Hauptfraktion.

Versuche, den Aldehyd im Vinylacetat zu binden und auf einfache Weise abzutrennen, sind mit den in der Tabelle aufgeführten Verbindungen unternommen worden, wobei in der letzten Spalte jeweils die niedrigsten erreichbaren Aldehydwerte aufgeführt sind. In Spalte 1 ist die Versuchsnummer, in Spalte 2 die Reaktionsbedingungen, in Spalte 3 das günstigste molare Verhältnis des Aldehyds zum Reagenz, in Spalte 4 die Ausgangskonzentration Acetaldehyd in ppm, in Spalte 5 die niedrigste erreichbare Endkonzentration (ppm) an Aldehyden im Vinylacetat angegeben.

0009812

Tabelle 1

| I | II | III | IV | V |
|---|---|---|---|---|
| 1 | 600 g VAc + 18 g Phenylhydrazin<br>30 Minuten Rückfluß | 1 : 3 | 3660 | 286 |
| 2 | 500 g VAc + 10 g Anilin<br>60 Minuten Rückfluß | 1 : 1,6 | 6124 | 820 |
| 3 | 600 g VAc + 18 g Harnstoff<br>60 Minuten Rückfluß | 1 : 3,9 | 5655 | 2847 |
| 4 | 100 g VAc + 50 ml 10 %-ige<br>wäßrige Harnstofflösung,<br>5 Minuten geschüttelt | 1 : 10 | 3660 | 824 |
| 5 | 600 g VAc + 18 g Hydrazinim-<br>dichlorid, 60 Minuten Rückfluß | 1 : 2,25 | 5655 | 8575 |
| 6 | 100 g VAc mit 50 g $NaHSO_3$-<br>Lösung, 10 %-ig in Wasser,<br>5 Minuten geschüttelt | 1 : 6 | 3660 | 0 |
| 7 | 60 g VAc mit 7,5 g $NaHSO_3$-<br>Lösung, 10 %-ig in Wasser,<br>5 Minuten geschüttelt | 1 : 1 | 5655 | 0 |

0009812

Die Tabelle zeigt, daß die Behandlung des aldehydhaltigen Vinylacetats mit Natriumhydrogensulfitlösung gute Resultate zeigt. Schon bei molarem Einsatz von Disulfit, bezogen auf Acetaldehyd in wäßriger Phase, wird der Acetaldehyd quantitativ in der wäßrigen Phase gebunden. Das behandelte Vinylacetat ist aldehydfrei. Alle anderen untersuchten Verbindungen zeigen diese Vorteile nicht.

Zur Durchführung des erfindungsgemäßen Verfahrens wird das Aldehyd enthaltende Vinylacetat mit wäßriger Natriumhydrogensulfitlösung vermischt. Dazu wird die 0,9 bis 1,2 molare Menge Alkalihydrogensulfit, vorzugsweise Natriumhydrogensulfit, pro Mol Aldehyd eingesetzt. Vorzugsweise können äquimolare Mengen Alkalihydrogensulfit, insbesondere Natriumhydrogensulfit, eingesetzt werden. Dies kann in Rührkesseln, durch gemeinsames Versprühen oder Vermischen in Mischrohren erfolgen. Das Natriumhydrogensulfit wird dabei vorzugsweise in verdünnter wäßriger Lösung eingesetzt, Konzentrationen zwischen 1 und 35 Gew.% sind üblich. Das Volumen der wäßrigen Phase soll dabei zwischen 0,5 und 35 % der Vinylacetatphase betragen. Neben Natriumhydrogensulfit können ggf. noch weitere Salze, z.B. Puffersalze für den Neutralbereich, Neutralsalze zur Verbesserung der Phasentrennung, wie z.B. Natriumchlorid, oder auch Entschäumer zugesetzt werden.

Der Übergang des Aldehyds in die wäßrige Phase erfolgt so schnell, daß bereits nach kurzzeitigem Rühren, z.B. einminütigem Vermischen der Vinylacetatphase mit der wäßrigen Phase, das Gemisch in einem Gefäß gesammelt werden kann, aus dem dann kontinuierlich bzw. diskontinuierlich die wäßrige Phase abgelassen werden kann. Das Vinylacetat kann dann der weiteren Verarbeitung zugeführt werden.

BAD ORIGINAL

Patentansprüche

1. Verfahren zur Abtrennung von Aldehyden, insbesondere Acetaldehyd, aus Vinylacetat, wobei das zu reinigende Vinylacetat mit bis zu $5 \times 10^4$ ppm Aldehyd, insbesondere Acetaldehyd, verunreinigt ist und das gereinigte Vinylacetat nach der Reinigung weniger als 50 ppm Aldehyd aufweist, d a d u r c h   g e k e n n z e i c h n e t , daß das zu reinigende Vinylacetat mit

Alkalihydrogensulfit in wäßriger Lösung, vorzugsweise der äquimolaren Menge Alkalihydrogensulfit in wäßriger Lösung, insbesondere Natriumhydrogensulfit in wäßriger Lösung, bezogen auf Aldehyd, durchmischt wird und nach erfolgter Phasentrennung die wäßrige Phase abgelassen wird.

2. Verfahren nach Anspruch 1, d a d u r c h   g e k e n n - z e i c h n e t , daß die Abtrennung der Aldehyde vom Vinylacetat diskontinuierlich erfolgt.

3. Verfahren nach Anspruch 1, d a d u r c h   g e k e n n - z e i c h n e t , daß die Abtrennung der Aldehyde vom Vinylacetat kontinuierlich erfolgt.

4. Verfahren nach Anspruch 1 bis 3, d a d u r c h   g e k e n n - z e i c h n e t , daß die Natriumhydrogensulfitlösung zusätzlich noch weitere anorganische Salze und/oder Entschäumer aufweist.

5. Verfahren nach Anspruch 1 bis 4, d a d u r c h   g e k e n n - z e i c h n e t , daß die wäßrige Phase nach der Phasentrennung einen pH zwischen 4 und 8 aufweist.

6. Verfahren nach Anspruch 1 bis 5, d a d u r c h   g e k e n n - z e i c h n e t , daß das Volumen der wäßrigen Phase 0,5 bis 35 % des Volumens des zu reinigenden Vinylacetats ausmacht.

7. Verfahren nach Anspruch 1 bis 6, d a d u r c h   g e k e n n - z e i c h n e t , daß die Natriumhydrogensulfitlösung in Wasser eine Konzentration zwischen 1 und 35 Gew.% aufweist.

Nummer der Anmeldung

EP 79103789.8

FÜR DEN RECHERCHENBERICHT

| EINSCHLÄGIGE DOKUMENTE | | | KLASSIFIKATION DER ANMELDUNG (Int.Cl.) 3 |
|---|---|---|---|
| Kategorie | Kennzeichnung des Dokuments mit Angabe, soweit erforderlich, der maßgeblichen Teile | betrifft Anspruch | |
| X | <u>DE - A - 1 618 238</u> (CELANESE CORPORATION)<br><br>÷ Ansprüche 1 bis 15; Seiten 3 bis 6, 9, 18; Beispiel +<br><br>-- | 1 - 7 | C 07 C 69/15<br>C 07 C 67/60 |
| | <u>DE - B - 2 252 276</u> (HOECHST)<br><br>÷ Beispiel +<br><br>-- | 1 | |
| | <u>DD - A - 126 327</u> (VEB LEUNA-WERKE " WALTER ULBRICHTE ")<br><br>÷ Beispiel 3 +<br><br>-- | 1 | |
| | FIESER, FIESER, "Reagents for Organic Synthesis", Bd. 1,<br><br>1967, JOHN WILEY AND SONS, INC., New York, London, Sydney, Seiten 1047 bis 1049<br><br>+ Seiten 1047 und 1049 +<br><br>---- | 1 | |

RECHERCHIERTE SACHGEBIETE (Int.Cl.) 3

C 07 C 69/00
C 07 C 67/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 14-12-1979 | KÖRBER |

EPA form 1503.1   06.78